# EUROPÄISCHE PATENTANMELDUNG

(11) **EP 4 306 956 A1**
(43) Veröffentlichungstag der Anmeldung: **17.01.2024**
(21) Anmeldenummer: 22185103.3
(22) Anmeldetag: 15.07.2022
(51) Int. Cl.: G01N 33/543, G01N 33/68

(54) **VERFAHREN ZUM IN-VITRO NACHWEIS ANTIGEN-SPEZIFISCHER IGE VERURSACHT DURCH EINE INFEKTION MIT PARASITEN ODER PILZEN**

(71) Anmelder: Justus-Liebig-Universität Gießen, 35390 Gießen (DE)
(72) Erfinder: Falcone, Franco, 35390 Gießen (DE); Prakash, Prema, 61231 Bad Nauheim (DE); Weber, Michael, 35039 Marburg (DE)
(74) Vertreter: Stumpf, Peter

(57) **Zusammenfassung**

Die Erfindung betrifft ein Verfahren zum in-vitro Nachweis einer parasitären oder pilzlichen Infektion unter Verwendung von infektionsspezifischen IgE aus Blutplasma oder Blutserum, die in nativer Form an eine Festphasenoberfläche gebunden werden und damit gestatten, infektiosspezifische Allergene in nativer Form, d.h. über die konformationellen Epitope selektiv zu binden und anschließend zu identifizieren.

## Beschreibung

Die vorliegende Erfindung betrifft ein Verfahren zur Beladung einer Festphasenoberfläche, die beladene Festphasenoberfläche selbst, sowie ihre Verwendung -je nach Ausprägung - zum in vitro Nachweis von parasitären oder pilzlichen Infektionen oder zur Identifikation, bzw. Auffindung und ggf. Isolierung, noch unbekannter Allergene, und damit auch von durch diese bisher unbekannten Allergene ausgelösten Allergien.

### Stand der Technik

Allergene sind Peptide oder Proteine, die allergische Reaktionen im menschlichen oder tierischen Körper auslösen. Eine Großzahl der Allergene ist diskontinuierlicher Natur, d.h. es gibt entlang der Proteinsequenz des Allergens mindestens zwei voneinander getrennte Sequenzabschnitte, die in der nativen Faltung des Proteins räumlich benachbarte Oberflächen-Bereiche bilden, die gemeinsam ein konformationelles Epitop bilden, anhand dessen das Allergen durch die körpereigenen Immunglobuline E (IgE) erkannt wird.

Im Stand der Technik werden unbekannte Allergene üblicherweise über ihre linearen Epitope identifiziert bzw. detektiert, z. B. mittels biochemischen Untersuchungsmethoden wie SDS-PAGE oder 2D-Gelelektrophorese. Identifikation bedeutet hierbei nicht zwingend die vollständige chemische Charakterisierung des Allergens. Dazu werden allergenhaltige Gemische wie etwa Proteinextrakte über zweidimensionale Gelelektrophorese (2D-Gelelektrophorese) aufgetrennt und die erhaltenen Blots mittels IgE-haltigen Seren auf IgE-bindende Proteine untersucht. Beide Schritte dieser sogenannten 2D-PAGE (isoelektrische Fokussierung und SDS-Polyacrylamid-Gelelektrophorese) sind denaturierende Techniken, bei denen diskontinuierliche Epitope (synonym: konformationelle Epitope) zerstört werden.

Eine weitere im Stand der Technik bekannte Methode verwendet sog. phage display libraries, bei denen Peptide unterschiedlicher Länge auf der Oberfläche von Phagen präsentiert werden, und im Rahmen eines wiederholten Screenings (sog. biopanning) zur Auswahl von Sequenzen führen, die an das IgE eines allergischen Individuums (Mensch oder Säugetier) führen. Durch bioinformatische Strukturvorhersagen lassen sich dadurch potentielle konformationelle Epitope auf einem bereits bekannten Allergen identifizieren.

Auch weitere im Stand der Technik bekannte Verfahren zur Identifikation konformationeller Epitope, d.h. diskontinuierlicher Epitope, auf Allergenen setzen voraus, daß das Allergen selbst bereits bekannt ist.

Eine weitverbreitete Methode zur Identifizierung diskontinuierlicher Epitope auf (bekannten) Allergenen ist zum Beispiel das sog. mimotope mapping. Hierzu wird ein für das zu untersuchende Allergen, dessen diskontinuierliche Epitope untersucht werden sollen, spezifisches IgE benötigt. Dieses spezifische IgE kann entweder monoklonaler Herkunft sein oder mittels des zu untersuchenden Allergens affinitätschromatographisch aus Serum aufgereinigt werden, was ein sehr aufwendiges Verfahren ist, und zudem voraussetzt, daß das Allergen an sich bereits bekannt ist. Die bekannten affinitätschromatographischen Verfahren weisen zudem den Nachteil auf, daß der an die stationäre Phase gebundene Ligand durch seine direkte Anbindung an die stationäre Phase konformativen Änderungen unterworfen wird, die die Bindungsaffinität zum gesuchten Analyten nachteilig beeinflussen. In einem Phagenexpressionssystem werden dann mit Hilfe dieses in Reinform verfügbaren IgE Peptide selektiert, die an die antigenbindende variable Region des IgE-Antikörpers binden. Die so identifizierten Peptidsequenzen sind in der Regel jedoch nicht mit den Epitopen auf den Allergenen identisch, insbesondere nicht bei Verwendung von (auch kommerziell erhältlichen) sog. Phage-display random peptide libraries, sondern ihnen nur ähnlich. Daher müssen nun noch bioinformatische Strukturvorhersage-Methoden eingesetzt werden, um - ausgehend von den identifizierten Phagen-präsentierten Sequenzen - zu ähnlich gefalteten Epitopen auf den Allergenen zu kommen. Das mimotope mapping gestattet nicht, neue (unbekannte) Allergene aufgrund ihrer diskontinuierlichen (konformationellen) Epitope zu identifizieren, da das Allergen für das Verfahren bereits bekannt sein muß, und entsprechendes spezifisches IgE bereits in Reinform vorhanden sein muß.

Eine weitere im Stand der Technik bekannte Möglichkeit, um neue Allergene zu identifizieren, ist die phage display-Technologie, die etwa bei der Identifikation der inhalatorischen Allergene in Kaffee eingesetzt wurde. Dabei wurde das spezifische IgE zweier Individuen mit inhalatorischer Kaffeeallergie mit Hilfe eines monoklonalen Maus-anti-human IgE Antikörpers für die Selektion bindender Proteine aus einer Phage display library identifiziert. Im Rahmen der Durchführung des Verfahrens ist der Aufbau einer entsprechenden umfangreichen Phage display cDNA library erforderlich, in der dann per Biopanning der Screening-Prozeß zur Auffindung des Allergens durchgeführt wird. Damit ist nun zwar ein Protein gefunden, das mit hoher Wahrscheinlichkeit das gesuchte Allergen ist, es muß jedoch noch verifiziert werden, daß dies wirklich der Fall ist. Dazu muß das so gefundene Protein noch monoklonal rekombinant hergestellt und gereinigt werden, um anschließend verifizierende Affinitätsmessungen an IgE durchzuführen (z. B. via ELISA-Test).

Diskontinuierliche Epitope bereits bekannter Allergene können natürlich auch via Röntgen-Kristallstrukturanalyse an Co-Kristallen von Allergen-Antikörperkomplexen identifiziert werden, in Verbindung mit zielgerichteter Mutagenese. Diese Methode ist aber nicht dazu geeignet, neue Allergene anhand ihrer nativen konformationellen Epitope zu identifizieren, da ja für die Röntgenstrukturanalyse entsprechende Co-Kristalle gezüchtet werden müssen - Allergen und Allergen-spezifisches IgE müssen also bereits bekannt und in ausreichender Menge und Reinheit verfügbar sein.

Die im Stand der Technik bekannten Verfahren zur Identifizierung neuer Allergene basieren auf Technologien wie Immunblotting, SDS-PAGE Gelelektrophorese oder 2D-PAGE, bei denen die dreidimensionale Struktur von Proteinen zerstört wird. Konformationelle Epitope werden dabei also zwangsläufig zerstört und in die einzelnen linearen (Teil-)Epitope aufgespalten, die dann - jedes für sich - mit diesen Verfahren identifiziert werden. Die Identifikation eines Allergens anhand seines nativen konformationellen Epitops kann so naturgemäß nicht gelingen. Bezüglich Lebensmittelallergien ist dies im Allgemeinen nicht nachteilig, da es sich bei ihnen üblicherweise um Allergene mit jeweils einem oder mehreren linearen Epitopen handelt. Die Epitope respiratorischer Allergene sind jedoch meist konformationeller (diskontinuierlicher) Natur.

Grundsätzlich können mit der Phage display Technologie zwar neue Allergene mit konformationellen Epitopen identifiziert werden, aber das Verfahren ist um ein Vielfaches aufwendiger und damit zeit- und kostenintensiver als das erfindungsgemäße Verfahren. Zudem besteht ein gravierender Nachteil (bzw. eine inhärente Unsicherheit) bei der Identifikation von konformationellen Epitopen mittels Phage display Technologie darin, daß posttranslationale Modifikationen, die native Allergene erfahren, und die Einfluß auf die Konformation der konformationellen Epitope haben, im Rahmen der rekombinanten Herstellung des Allergens grundsätzlich nicht realisiert werden können. Dieses Verfahren bietet daher keine Sicherheit, daß ein konformationelles Epitop sicher identifiziert wurde, und bedarf daher immer verfahrensunabhängiger Nachprüfung.

Resultierend aus den vorstehend beschriebenen mangelhaften Möglichkeiten für die Identifikation konformationeller Epitope im Stand der Technik, insbesondere den aufwendigen und dennoch unzureichenden Möglichkeiten, die Interaktion zwischen Allergen und Allergen-spezifischem IgE möglichst nativ zu realisieren, besteht zwangsläufig auch der Wunsch nach einem auf der möglichst nativen Interaktion von Allergen und Allergen-spezifischem IgE basierenden sicheren, schnellen und kostengünstigen Nachweis einer entsprechenden Infektion. Insbesondere die Spezifität des Nachweises einer parasitären oder pilzlichen Infektion steigt naturgemäß mit zunehmendem Grad an nativer Wechselwirkung zwischen dem Allergen und dem Allergen-spezifischen IgE.

Es besteht somit großer Bedarf sowohl an einem Verfahren für die Identifikation neuer, bislang unbekannter, konformationeller Epitope, das die vorstehend beschriebenen Nachteile nicht aufweist, als auch an einem sicheren und verlässlichen Diagnoseverfahren für parasitäre oder pilzliche Infektionen (sowie auch Allergien), basierend auf einer möglichst nativen Interaktion zwischen Allergen-spezifischem IgE und Allergen.

### Aufgabe

Aufgabe des Gegenstands der Erfindung ist es einerseits, parasitäre oder pilzliche Infektionen zu diagnostizieren (nachzuweisen) sowie andererseits unbekannte Allergene anhand ihrer natürlichen Konformation (via sog. konformationeller Epitope) schnell, sicher und kostengünstig zu identifizieren. Beide Aufgaben hängen eng miteinander zusammen, da sie beide auf der selektiven Wechselwirkung von oberflächenfixiertem Allergen-spezifischen IgE mit beispielsweise parasitären oder pilzlichen Allergenen beruhen, wobei die Wechselwirkung im wesentlichen der natürlichen Wechselwirkung entspricht. Zentraler Kern des Gegenstands der Erfindung ist daher ein Verfahren zur Bereitstellung einer mit IgE beladenen Festphasenoberfläche, bzw. die IgE-beladene Festphasenoberfläche selbst, wobei die IgE-Moleküle bzw. Molekülkomplexe trotz der Fixierung an der Festphasenoberfläche im wesentlichen in ihrer nativen Konformation verbleiben, so daß sie unter im wesentlichen natürlichen Bedingungen mit konformationellen Epitopen interagieren, wodurch es ermöglicht wird, natürliche oder chemisch modifizierte Allergene anhand ihrer konformationellen Epitope, die mit den IgE wechselwirken, an die IgE-beladene Festphasenoberfläche zu binden. Dem Fachmann ist die Bedeutung des Begriffs beladene Festphasenoberfläche bekannt. Synonym kann aus chemischer Sicht auch von einer beschichteten Festphasenoberfläche gesprochen werden, wobei die Anhaftung sowohl physikalischer als auch chemischer Natur sein kann, entweder in Kombination miteinander oder auch jeweils ausschließlich. Außerdem kann eine Schicht bzw. Beladung auf einer Festphasenoberfläche durch eine Zwischenschicht, oder auch mehrere Zwischenschichten, vermittelt sein. Als weiteres Synonym wird auch der Begriff modifizierte Festphasenoberfläche verwendet, z. B. IgEmodifizierte Festphasenoberfläche. Das für die Beladung/Beschichtung/Modifizierung vorgesehene Protein (IgE) stammt - je nach Ausführungsvariante des Gegenstands der Erfindung - entweder aus einem menschlichen oder tierischen Patienten oder einer Umweltprobe, bezüglich derer der Verdacht besteht, daß sie das noch unbekannte Allergen enthält. Bei tierischen Patienten handelt es sich grundsätzlich um Säugetiere wie beispielsweise Hunde, Katzen, Pferde, Rinder, Schafe und viele andere mehr, da nur Säugetiere über IgEs verfügen. Nachfolgend sind mit dem allgemeinen Begriff Patient immer entweder tierische oder menschliche Patienten gemeint.

### Lösung der Aufgabe

Die zentrale Aufgabe der Durchführung der Wechselwirkung von Allergen und Allergen-spezifischem IgE unter im wesentlichen natürlichen Bedingungen an einer Festphasenoberfläche, d.h. die Bindung von Allergenen in nicht-denaturierter Form, wird erfindungsgemäß gelöst durch die Anbindung von Allergen-spezifischem IgE an eine Festphasenoberfläche unter Verwendung des humanen IgE Rezeptors FcεRIα-(His)₆. Dieser hochaffine IgE Rezeptor weist sog. Histidin-Tags ((His)₆) auf und ist bevorzugt durch ein rekombinantes Herstellungsverfahren hergestellt. Durch die Verwendung dieses IgE-Rezeptors als Bindeglied/Anker/Linker - oder zumindest als Teil eines solchen - zwischen Festphasenoberfläche und IgE wird eine IgEmodifizierte Festphasenoberfläche erhalten, auf der das Allergen-spezifische IgE, sowie ggf. weitere IgE-Varianten, in im wesentlichen nativem Zustande vorliegen. Ausgehend von der so erhaltenen IgE-modifizierten Festphasenoberfläche können je nach Ausgestaltung verschiedene Teilaufgaben gelöst werden.

Die Beladung/Beschichtung der Festphasenoberfläche mit dem humanen IgE Rezeptor FcεRIα-(His)₆ und damit auch über diesen Anker mit IgE sowie Allergen muß nicht zwingend vollständig, d.h. zu 100% der möglichen Beladung, erfolgen. Es ist erfindungsgemäß, diese Beladung/Beschichtung der Festphasenoberfläche auch nur bereichsweise vorzunehmen, so daß Beladungsgrade der Festphasenoberfläche in einem weiten Bereich von 1% Beladung bis zu 100% Beladung möglich und erfindungsgemäß sind.

Nachfolgend werden zwei Ausgestaltungen der Erfindung beschrieben, ohne daß damit eine Einschränkung des Gegenstands der Erfindung darauf verbunden ist.

Bei beiden Ausgestaltungen stammt das für die Beladung der Festphasenoberfläche verwendete IgE jeweils aus einer (Blut-)Serumprobe, bzw. wird daraus an die Festphasenoberfläche gebunden, ohne daß damit eine Einschränkung verbunden ist. Das IgE kann auch aus einer (Blut-)Plasmaprobe heraus an die Festphasenoberfläche gebunden werden, sofern die Gerinnung der (Blut-)Plasmaprobe in geeigneter Weise erfolgt ist, etwa durch Zusatz von Heparin oder anderen gerinnungshemmenden Mitteln, die Metallatome nicht komplexieren. Dem Fachmann ist weiterhin bekannt, daß IgE nicht nur in Serum- oder Plasma-Proben enthalten ist, sondern auch in anderen geeignet vorbereiteten Proben, etwa solchen aus Biopsien, wie z.B. zerebrospinale Flüssigkeit, Speichel oder Tränenflüssigkeit. Wenn daher nachfolgend von Serum gesprochen wird, ist immer implizit auch als Alternative Plasma mit gemeint sowie auch andere geeignet aufbereitete Proben, z. B. aus Bioptaten. Als allgemeiner übergeordneter Begriff, der alle vorgenannten sowie auch alle weiteren dem Fachmann bekannten IgE-haltigen Proben mit umfasst, wird nachfolgend auch der Begriff *biologische Probe* verwendet.

Die grundsätzliche (primäre) Aufgabe wird gelöst durch den Hauptanspruch 1. Von Hauptanspruch 1 abgeleitete Nebenansprüche stellen vorteilhafte Ausgestaltungen des Gegenstands der Erfindung dar.

Die (Teil-)Aufgabe der Diagnose von parasitären oder pilzlichen Infektionen als eine Verwendung der erfindungsgemäß proteinbeschichteten Festphasenoberfläche wird erfindungsgemäß gelöst durch die kennzeichnenden Merkmale von Anspruch 3. Die von Anspruch 3 abhängigen Ansprüche stellen weitere vorteilhafte Ausgestaltungen des Gegenstands der Erfindung dar.

Die (Teil-)Aufgabe der schnellen, sicheren und kostengünstigen Identifikation unbekannter Allergene anhand ihrer natürlichen Konformation (als sog. konformationelle Epitope) als eine weitere Verwendung der erfindungsgemäß proteinbeschichteten Festphasenoberfläche wird erfindungsgemäß gelöst durch die kennzeichnenden Merkmale von Anspruch 2. Die von Anspruch 2 abhängigen Ansprüche stellen weitere vorteilhafte Ausgestaltungen des Gegenstands der Erfindung dar.

Werden aus Serumproben, Bioptaten oder anderen Proben eines Patienten, der an einer Allergie leidet, deren Ursache noch nicht bekannt ist, möglichst sämtliche Varianten des Immunglobulins E (IgE), die in der Serumprobe enthalten sind, an eine geeignete Festphasenoberfläche gebunden, d.h. die Festphasenoberfläche mit ihnen beladen, so ist auf dieser IgE-beschichteten Festphasenoberfläche dann zwangsläufig auch die mindestens eine IgE-Variante gebunden, die das die Allergie auslösende Allergen bindet. Diese IgE-beschichtete Festphasenoberfläche kann somit verwendet werden, um das noch unbekannte Allergen in seiner natürlichen, physiologischen Form zu isolieren, denn das betreffende noch unbekannte Allergen bindet spezifisch an das entsprechende IgE aufgrund ihrer beider natürlichen Affinitäten zueinander. Ursache dieser beiden natürlichen Affinitäten zueinander sind Wechselwirkungen zwischen dem IgE und dem Allergen, die dem Fachmann unter dem Sammelbegriff Schlüssel-Schloß-Prinzip bekannt sind. Die IgE-beschichtete Festphase wird somit als Sensor für Screening-Tests mit verschiedensten potentiellen Allergenextrakten verwendet, der das gesuchte konformationelle Epitop als solches erkennt und bindet. Nach Waschen der IgE+Allergen-beschichteten Festphasenoberfläche und Ablösen der von den IgE gebundenen Allergene können diese erfindungsgemäß in klassischer Weise analysiert und das unbekannte allergieauslösende Allergen mit Hilfe von Datenbank-Recherche bekannter Allergene nach dem Ausschlußprinzip identifiziert werden.

Bei dem erfindungsgemäßen Verfahren zur Identifikation noch unbekannter Allergene anhand ihrer konformationellen Epitope gelingt dies insbesondere dadurch, daß diese Allergene in ihrem nativen, d.h. natürlichen Zustand isoliert und damit letztendlich nachgewiesen werden. Erreicht wird dies zum einen dadurch, daß die Bindung der IgE-Varianten aus der Serumprobe an die Festphasenoberfläche durch den IgE Rezeptor FcεRIα-(His)₆ erfolgt, was dazu führt, daß die IgE-Varianten - und damit auch die Allergen-spezifische Variante - trotz Bindung an die Festphasenoberfläche weitestgehend in ihrer nativen Form verbleiben. Bei direkter Anbindung der IgE an die Festphasenoberfläche unvermeidliche, die native IgE-Konformation störende, Wechselwirkungen zwischen der Festphasenoberfläche und dem IgE werden so vermieden. Diese unerwünschten -weil die native IgE-Konformation störenden Wechselwirkungen der Festphasenoberfläche sind insofern vergleichbar der dem Fachmann bekannten allosterischen Hemmung von Enzymen: Dabei führt die Interaktion eines Hemmstoffes mit einem Enzym, die nicht an der aktiven Position des Enzyms erfolgt, zu konformationellen Änderungen des Enzyms an der aktiven Position, so daß das Enzym gehemmt wird. Analoge Effekte der Festphasenoberfläche auf das IgE, die zu einer mehr oder weniger starken Inaktivierung des IgE führen würden, wenn es direkt an die Festphasenoberfläche gebunden wäre, werden durch die erfindungsgemäße Verwendung des IgE Rezeptors FcεRIα-(His)₆ als Ankermolekül vermieden. Weiterhin werden auch die Allergene in ihrem nativen, natürlichen Zustand verwendet (infolge der direkten Bindung aus der Probe heraus), in welchem sie alle natürlicherweise vorliegenden post-translationalen Modifikationen aufweisen, wie beispielsweise Glykosylierungen, Phosphorylierungen, Acetylierungen, partielle Proteolyse, Disulfidbrücken.

Neben der vorstehend beschriebenen Aufgabe, unbekannte Allergene anhand ihrer nativen Form, also anhand ihrer konformationellen Epitope, zu erkennen, kann der hier offenbarte Gegenstand der Erfindung auch die Aufgabe lösen, Antikörper gegen bekannte Allergene, insbesondere parasitäre oder pilzliche Allergene, nachzuweisen und damit eine entsprechende Infektion zu diagnostizieren:
Die für dieses extrakorporale Diagnoseverfahren erforderlichen Verfahrensschritte sind in wesentlichen Teilen identisch zu den Verfahrensschritten, die erforderlich sind, um unbekannte Allergene zu identifizieren, bzw. zu detektieren. Die Identität betrifft insbesondere die Verfahrensschritte bis zur Bereitstellung der IgE-beschichteten Festphasenoberfläche, die dadurch erreicht wird, daß in beiden Fällen als Ankermolekül bzw. Linker für die Bindung der IgE an die Festphasenoberfläche der IgE Rezeptor FcεRIα-(His)₆ verwendet wird, und das für die jeweilige Ausgestaltung erforderliche IgE in seiner nativen Form aus einer biologischen Probe stammt.

Bei dem extrakorporalen Diagnoseverfahren werden aus einer biologischen Probe eines Patienten, der an einer Allergie leidet, deren Ursache in diesem Falle jedoch bereits bekannt ist, möglichst sämtliche Varianten des Immunglobulins E (IgE), die in der biologischen Probe enthalten sind, an eine geeignete Festphasenoberfläche gebunden. Auf dieser IgE-beschichteten Festphasenoberfläche ist zwangsläufig auch die mindestens eine IgE-Variante gebunden, die das die Infektion begleitende, bereits bekannte, parasitäre oder pilzliche Allergen bindet. Mit dem Nachweis dieser mindestens einen gesuchten IgE-Variante auf dieser IgE-beschichteten Festphase ist dann indirekt zwangsläufig auch die entsprechende parasitäre oder pilzliche Infektion zum Zeitpunkt oder kurz vor dem Zeitpunkt der Probennahme der biologischen Probe bei dem Patienten nachgewiesen, von dem die biologische Probe genommen wurde. Der Nachweis der mindestens einen IgE-Variante, die mit einer parasitären oder pilzlichen Infektion ursächlich verbunden ist, erfolgt, indem die IgE-beschichtete Festphase mit mindestens einem markierten parasitären und/oder pilzlichen Allergen, das - abgesehen von der Markierung - in seiner ansonsten natürlichen, physiologischen Form vorliegt, inkubiert wird, denn das betreffende bekannte Allergen, das als die Infektion begleitend vermutet wird, bindet spezifisch an das entsprechende IgE. Es ist lediglich erforderlich, nach einem vorbereitenden Waschschritt die (bei positivem Ergebnis) IgE+Antigen-beschichtete Festphase daraufhin zu untersuchen, ob das die Infektion begleitende Antigen an die IgE-beschichtete Festphase gebunden ist. Dieser Nachweis erfolgt erfindungsgemäß mit beliebigen im Stand der Technik bekannten Nachweisverfahren auf die mindestens eine Markierung (z. B. fluorometrisch, radiometrisch), die das mindestens eine hier als Reagenz verwendete bekannte parasitäre oder pilzliche Allergen aufweist.

Bei dem erfindungsgemäßen Verfahren zum Nachweis einer parasitären oder pilzlichen Infektion anhand der konformationellen Epitope des parasitären oder pilzlichen Allergens gelingt dies insbesondere dadurch, daß diese Allergene als Reagenzien in möglichst nativem, d.h. natürlichen Zustand eingesetzt werden, verbunden mit dem im wesentlichen nativen Zustand der an die Festphasenoberfläche gebundenen IgE, was dadurch erreicht wird, daß die Bindung der IgE-Varianten aus der Serumprobe an die Festphasenoberfläche durch den IgE Rezeptor FcεRIα-(His)₆ erfolgt. Durch die Verwendung dieses Rezeptors als Ankermolekül verbleiben die IgE trotz Bindung an die Festphasenoberfläche weitestgehend in ihrer nativen Form (siehe oben). Die Wahrung der im wesentlichen nativen Form der markierten Allergene als Reagenzien wird erreicht, indem eine Markierung ausgewählt wird, die die natürliche Konformation des konformationellen Epitops möglichst wenig beeinträchtigt, beispielsweise dadurch, daß eine Fluoreszenzmarkierung an einer Position des Allergens angebracht wird, wo der Fluoreszenz-Chromophor die räumliche Struktur möglichst wenig beeinträchtigt. Dies kann i.d.R über die Verwendung sogenannter flexibler Linkersequenzen gewährleistet werden.

Die beiden Ausgestaltungen des Verfahrens werden nachfolgend beispielhaft im Detail beschrieben, ohne daß damit eine Einschränkung des Gegenstands der Erfindung auf die offenbarten Ausführungsbeispiele verbunden ist. So umfassen beispielsweise Bereichsangaben stets alle - auch nicht explizit genannte - innerhalb der genannten Intervallgrenzen liegenden Zwischenwerte und alle denkbaren Teilintervalle.

### A) Ausgestaltung als Verfahren zur Identifikation eines noch unbekannten Allergens

Eine geeignete Festphasenoberfläche wird mit dem IgE Rezeptor FcεRIα-(His)₆ beschichtet, wodurch eine entsprechende FcεRIα-(His)₆-beschichtete Festphasenoberfläche erhalten wird, d.h. eine Festphasenoberfläche, an die FcεRIα-(His)₆ gebunden ist. Die Bindung des FcεRIα-(His)₆ an die Festphasenoberfläche kann sowohl auf chemischem Wege, d. h. durch eine oder mehrere chemische Bindungen erfolgen (ionisch, kovalent, komplexartig), als auch auf physikalischem Wege, d.h. rein adsorptiv durch Adsorptionskräfte. Es ist auch möglich, daß eine Kombination aus chemischer Bindung und adsorptiver Bindung vorliegt. Außerdem ist es möglich und ebenfalls erfindungsgemäß, daß die Bindung des IgE Rezeptors FcεRIα-(His)₆ an die Festphasenoberfläche indirekt erfolgt, d.h. durch einen sog. "Anker" vermittelt. Dem Fachmann sind geeignete "Anker"-Moleküle bzw. "Anker"-Strukturen bekannt, die erfindungsgemäß eingesetzt werden können, zum Beispiel Ni²⁺-NTA. Auch die Bindung des Ankers an die Festphasenoberfläche und die Bindung des FcεRIα-(His)₆ an den Anker kann jeweils auf die verschiedenen vorstehend beschriebenen Arten erfolgen.

Geeignete erfindungsgemäß verwendbare Festphasenoberflächen sind dem Fachmann bekannt. Beispielhaft seien genannt: Ni-NTA-Partikel, sog. Ni²⁺-NTA magnetische beads. Die Verwendung derartiger magnetischer Partikel hat den Vorteil, daß diese mobil sind, d.h. durch Anlegen geeigneter Magnetfelder können diese sowohl bewegt als auch fixiert werden, je nach gerade durchzuführendem Verfahrensschritt. Insbesondere Separations- und Reinigungsschritte sind mittels magnetischer Partikel sehr effizient durchführbar. Im Falle der Verwendung von Partikeln ist die erfindungsgemäß geeignete Festphasenoberfläche somit gebildet durch die Gesamtheit der einzelnen Partikeloberflächen, d.h. die Summe aller Einzelpartikeloberflächen bildet die erfindungsgemäße Festphasenoberfläche.

Erfindungsgemäß kann die Festphasenoberfläche aber auch in einem Stück ausgebildet sein, etwa in Form eines mehr oder weniger kompakten Sinterkörpers, einer Polymermembran, eines Filtervlieses, einer Metalloberfläche oder einem anderen makroskopischen Körper, bzw. Körper-Verbund (etwa ein Filtervlies). Hierbei handelt es sich dann um eine stationäre Festphasenoberfläche, die ihrerseits vorteilhafte Verwendungseigenschaften aufweist, etwa die, daß sie bereichsspezifisch erfindungsgemäß modifiziert/beschichtet werden kann.

Die wie vorstehend beschrieben erhaltene FcεRIα-(His)₆-beschichtete Festphasenoberfläche wird mit einer Serumprobe bzw. biologischen Probe inkubiert, wobei sich das in der biologischen Probe enthaltene IgE an das auf der Festphasenoberfläche befindliche FcεRIα-(His)₆ bindet und somit auf die Festkörperoberfläche fixiert wird. Man erhält somit eine IgE-beschichtete Festphasenoberfläche, wobei die IgE-Beschichtung auf der Festphasenoberfläche aus im wesentlichen allen in der biologischen Probe ursprünglich enthaltenen IgE-Varianten besteht, da die Bindungskraft zwischen FcεRIα-(His)₆ und IgE unabhängig von der spezifischen Variante des IgE sehr groß ist. Unter allen auf der Festphasenoberfläche fixierten IgE-Varianten ist - im Falle eines positiven Verfahrensergebnisses - auch das Allergen-spezifische IgE befindlich, das für den nachfolgenden Verfahrensschritt, der Identifikation, essentiell ist.

Nach der Inkubation mit der biologischen Probe wird zunächst ein Waschschritt durchgeführt, um die nun von den IgE befreite biologische Probe sowie alle bei der Identifikation evtl. störenden weiteren Inhaltsstoffe der biologischen Probe zu entfernen (z. B. IgG, IgM, Albumin u.a.). Auf der Festphasenoberfläche verbleiben somit lediglich die Allergen-spezifischen IgE. Dem Fachmann sind erfindungsgemäß einsetzbare Waschprozeduren - sowohl bei Verwendung von mobilen Festphasenoberflächen als auch bei Verwendung von stationären Festphasenoberflächen - wohlbekannt, so daß darauf nicht näher eingegangen werden muß. Prinzipiell kann nach jedem Verfahrensschritt ein Waschschritt vorgesehen werden.

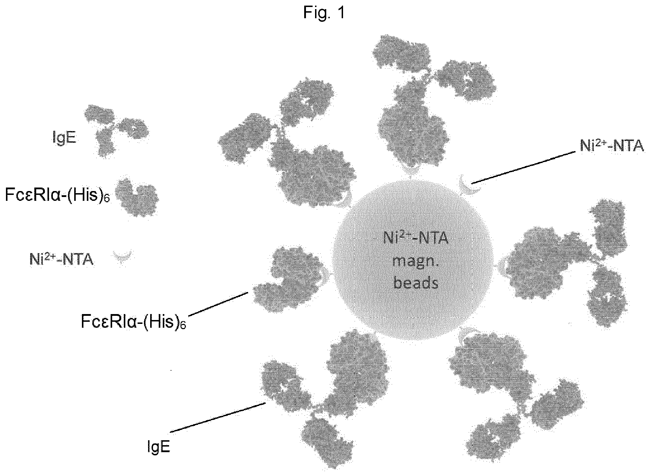

Durch die Verwendung von FcεRIα-(His)₆ als Anker zur Fixierung von IgE entspricht die Konformation des auf der Festkörperoberfläche fixierten IgE im wesentlichen der nativen Form, da die Bindung des IgE an das FcεRIα-(His)₆ der natürlichen Bindung entspricht, wodurch die IgE-Konformation nicht durch die Bindung an die Festphasenoberfläche beeinträchtigt wird. Das FcεRIα-(His)₆ schützt das IgE somit vor nachteiligen Einflüssen der Festphasenoberfläche auf die native Konformation und bewahrt diese somit trotz der Bindung an die Festphasenoberfläche (vgl. Fig. 1).

Wenn die biologische Probe von einem Patienten stammt, der an einer Allergie leidet, deren Ursache (auslösendes Allergen) nicht bekannt ist, so stellt die IgE-beschichtete Festphasenoberfläche nun ein Allergen-Adsorptionsmittel dar, das im weiteren Verlauf des Verfahrens verwendet wird, um das unbekannte Allergen an der IgE-beschichteten Festphasenoberfläche zu fixieren.

Dazu wird die IgE-beschichtete Festphasenoberfläche mit Allergenextrakten inkubiert, deren Herkunft bekannt ist und von denen vermutet wird, daß sie das gesuchte Allergen enthalten. Optimalerweise werden die Allergenextrakte unter physiologischen Bedingungen hergestellt und nach Möglichkeit auch die Inkubation der IgE-beschichteten Festphasenoberfläche mit den Allergenextrakten unter physiologischen Bedingungen durchgeführt, um möglichst weitgehend sicherzustellen, daß das gesuchte Allergen in seiner nativen Form mit dem auf der Festphasenoberfläche fixierten nativen IgE in Kontakt kommt. Zwingend erforderlich ist die Allergenextraktgewinnung unter physiologischen Bedingungen jedoch nicht, da Allergene ja auch natürlicherweise harsche Umgebungsbedingungen (reaktive Sauerstoffspezies in der Umgebungsluft, Licht-/UV-Bestrahlung u. dgl.) ertragen, bevor sie in den empfindlichen Organismus gelangen. Beispiele für Allergenextrakte sind generell komplexe Proteinextrakte wie etwa Pollenextrakte oder Zellenextrakte.

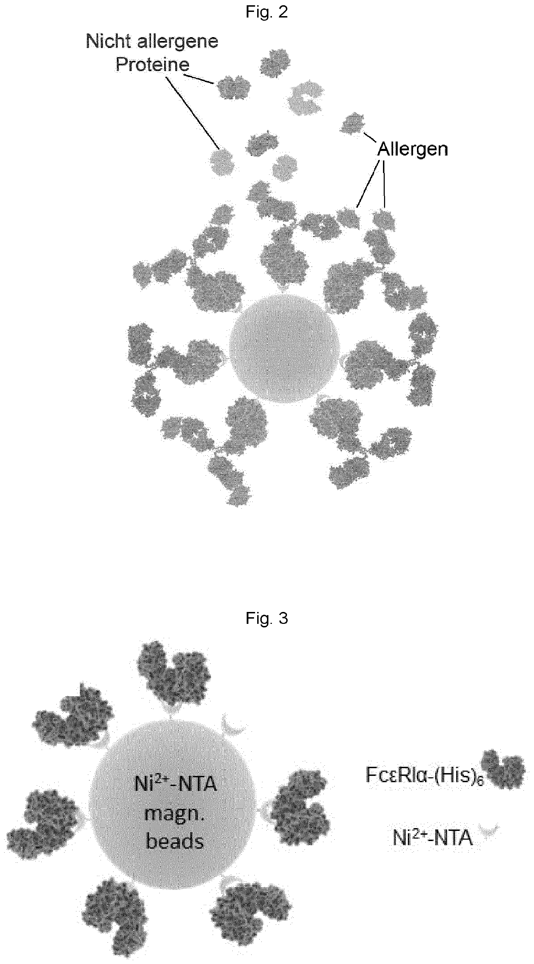

Im Rahmen dieses Allergenextrakt-Screenings werden von dem auf der Festphasenoberfläche befindlichen Allergen-spezifischen IgE nur das in einem Allergenextrakt befindliche spezifische Allergen gebunden, das die allergische Reaktion auslöst, denn durch dieses ist das Festphasen-gebundene Allergen-spezifische IgE in dem allergisch reagierenden Patienten gebildet worden. Durch einen oder mehrere geeignete Waschschritte werden nun alle nicht-Allergene sowie ungebundenen (nicht adsorbierten) Proteine entfernt, und es verbleibt eine Allergen-beschichtete Festphasenoberfläche, auf der sich nur das gesuchte (aber noch unbekannte) Allergen befindet, da dieses von dem Allergen-spezifischen IgE gebunden ist, das seinerseits durch das FcεRIα-(His)₆ an die Festphasenoberfläche gebunden ist, sofern der geprüfte Allergenextrakt das betreffende Allergen enthalten hat (vgl. Fig. 2).

Da dem Fachmann bekannt ist, welcher Allergenextrakt mit der IgE-beschichteten Festphasenoberfläche inkubiert wurde, ist nach entsprechender Untersuchung der Allergenextrakt-inkubierten und gewaschenen Festphasenoberfläche das Allergen und dessen Herkunft bekannt.

Für diese Untersuchung, also die eigentliche Identifikation des Allergens wird dieses vorzugsweise zunächst von der Festphasenoberfläche abgelöst. Dem Fachmann sind dazu geeignete Wasch-Prozeduren bekannt, die sämtlich erfindungsgemäß eingesetzt werden können. Beispielhaft sei die Elution mit Imidazol-Puffer genannt, wodurch FcεRIα-(His)₆, IgE und Allergen beispielsweise von einer Ni-NTA-Oberfläche abgelöst werden. Es ist nicht zwingend erforderlich, daß FcεRIα-(His)₆, IgE und Allergen von der Festphasenoberfläche abgelöst werden, sondern sogar vorteilhaft, wenn lediglich das gesuchte Allergen von der beschichteten und inkubierten Festphasenoberfläche abgelöst wird, und FcεRIα-(His)₆ sowie IgE darauf verbleiben, denn dann enthält das so erhaltene Eluat weniger Begleitstoffe, die die nachfolgende Identifizierung stören oder verkomplizieren können.

Das durch den vorstehenden Elutionsschritt erhaltene und - im Falle eines positiven Testergebnisses - allergenhaltige Eluat wird nun analysiert, wobei es bei dieser Analyse nun nicht mehr notwendig ist, daß das Allergen in seiner nativen Form verbleibt, denn es wurde ja bereits in seiner nativen Form selektiv an die IgE-beschichtete Festphasenoberfläche gebunden, wodurch die Isolierung unter selektiver Bindung via konformationellem Epitop bereits erfolgt ist. Damit ist bereits sichergestellt, daß es sich um das korrekte Allergen handelt. Für die Identifikation, bzw. Bestimmung des Allergens kann daher erfindungsgemäß nun jedes der bereits im Stand der Technik bekannten Verfahren zur Identifikation/Bestimmung eines Allergens verwendet werden. Konkret beispielhaft genannt seien hier als eine Verfahrensvariante die Auftrennung via SDS-PAGE oder 2D-Gelelektrophorese mit anschließender massenspektrometrischer Identifikation in Verbindung mit datenbankgestützter Aussortierung solcherart bereits bekannter Allergene. Dieses hier beispielhaft als letzter Verfahrensschritt genannte eigentliche Identifikationsverfahren ist nicht einschränkend zu verstehen.

Diese Ausgestaltung des Gegenstands der Erfindung als ein Verfahren zur Identifizierung eines noch unbekannten Allergens anhand seines konformationellen Epitops kann somit wie folgt als ein Verfahren zusammengefasst werden, das die folgenden Verfahrensschritte sowie ggf. Ergänzungen umfasst:
i) Bereitstellung mindestens einer Festphasenoberfläche, die zumindest bereichsweise mit dem IgE Rezeptor FcεRIα-(His)₆ beladen ist, wobei die IgE-Bindungsstelle des IgE Rezeptors FcεRIα-(His)₆ in der Lage ist, IgE zu binden.
ii) Inkubierung der gemäß Schritt i) bereitgestellten mindestens einen Festphasenoberfläche mit einer biologischen Probe, welche im Falle einer allergischen Reaktion des Patienten, von dem die biologische Probe stammt, auch mindestens ein IgE enthält, das das mindestens ein unbekanntes Allergen bindet, das im Falle einer Allergie unbekannter Ursache mindestens in der biologischen Probe vorhanden ist, wobei der an die Festphasenoberfläche gebundene IgE Rezeptor FcεRIα-(His)₆ seinerseits die in der biologischen Probe enthaltenen IgE bindet, so daß die aus der biologischen Probe stammenden IgE über den IgE Rezeptor FcεRIα-(His)₆ an die Festphasenoberfläche gebunden werden, wodurch eine Festphasenoberfläche erhalten wird, die vermittelst FcεRIα-(His)₆ als Anker mit IgE aus einer biologischen Probe beladen ist.
iii) Waschen der gemäß Schritt ii) erhaltenen, mindestens einen mit IgE beladenen Festphasenoberfläche.
iv) Durchführung eines Allergen-Screenings, indem mindestens eine der gemäß Schritt iii) erhaltenen mit IgE beladenen Festphasenoberfläche mit mindestens einem Allergenextrakt bekannter Herkunft inkubiert wird, so dass das allergieauslösende Allergen, sofern es in dem mindestens einen Allergenextrakt bekannter Herkunft enthalten ist, über das dieses Allergen bindende IgE an die Festphasenoberfläche gebunden wird, was nur dann erfolgt, wenn das dieses Allergen bindende IgE in der zu untersuchenden biologischen Probe während der Durchführung von Schritt ii) vorhanden war.
v) Waschen der gemäß Schritt iv) inkubierten mindestens einen Festphasenoberfläche, so dass nicht gebundene störende Begleitstoffe aus dem mindestens einen Allergenextrakt bekannter Herkunft entfernt werden, wodurch eine Festphasenoberfläche erhalten wird, die das über das konformationelle Epitop gebundene noch unbekannte Allergen aufweist, sofern der entsprechende Allergenextrakt bekannter Herkunft das Allergen enthalten hat.
vi) Untersuchung der gemäß Schritt v) gewaschenen Festphasenoberfläche mittels eines Verfahrens, das das Allergen nachweist, wobei nur bei der mindestens einen Festphasenoberfläche ein positives Untersuchungsergebnis erhalten wird, die mit dem allergieauslösenden Allergenextrakt bekannter Herkunft inkubiert wurde, wodurch das entsprechende Allergen anhand seines konformationellen Epitops isoliert und anschließend nachgewiesen wurde.

Die Untersuchung gemäß vorstehendem Schritt vi) zur finalen Identifikation kann die Abtrennung des in nativer Form gebundenen Allergens von der Festphasenoberfläche umfassen, beispielsweise durch Elution mit Imidazol-Puffer, wodurch der IgE Rezeptor FcεRIα-(His)₆, IgE und das gesuchte Allergen von der Festphasenoberfläche abgelöst werden. Nach dieser Abtrennung kann das Allergen dann erfindungsgemäß mit Verfahren identifiziert werden, die im Stand der Technik bekannt sind, etwa Auftrennung des Eluats via SDS-PAGE oder 2D-Gelelektrophorese mit anschließender Identifikation mittels Verfahren der Proteomik und/oder Massenspektrometrie. Hierbei ist es nicht erforderlich, die native Form des Allergens aufrechtzuerhalten, da die Isolierung per Bindung an das entsprechende IgE ja bereits in nativem Zustande erfolgt ist.

Bei der Festphasenoberfläche kann es sich um eine makroskopische stationäre Festphase handeln, die entweder monolithisch ausgebildet ist oder aus einem Faserverbund besteht. Diese Festphasenoberfläche kann aus einem keramischen und/oder einem polymeren Material bestehen.

Bei der Festphasenoberfläche kann es sich um eine Festphasenoberfläche handeln, die aus der Gesamtheit der Partikeloberflächen einer Menge von Partikeln besteht, die gemeinsam den Verfahrensschritten i) bis vi) sowie ggf. weiteren sich daran anschließenden Schritten unterzogen werden. Bei den Partikeln kann es sich um magnetische Partikel handeln.

### B) Ausgestaltung als Verfahren zum Nachweis einer parasitären oder pilzlichen Infektion

Das erfindungsgemäße Verfahren zum Nachweis einer parasitären oder pilzlichen Infektion wird extrakorporal durchgeführt, d.h. nicht am Patienten. Anwendbar ist es beispielsweise für Infektionen durch die Pilze der Gattungen Alternaria, Cladosporium, Penicillium, Aspergillus, Malassezia, wobei diese Auflistung nicht als einschränkend zu verstehen ist.

Die ersten Verfahrensschritte des erfindungsgemäßen Verfahrens in der Ausgestaltung als Nachweisverfahren einer parasitären Infektion sind identisch mit den ersten Verfahrensschritten in der Ausgestaltung zur Identifikation eines noch unbekannten Allergens. In beiden Varianten des Gegenstandes der Erfindung wird eine IgE-beschichtete Festphasenoberfläche bereitgestellt, wobei es sich bei den IgE-Molekülen bzw. Molekülkomplexen um solche handelt, die aus einer biologischen Probe eines Patienten gewonnen wurde, der entweder an einer Allergie leidet, die von einem noch nicht bekannten Allergen hervorgerufen wird (Variante A - Allergen-Identifikation), oder bei dem der Verdacht einer Parasiten-Infektion besteht (Variante B - Infektionsnachweis) - vgl. dazu die Abbildungen Fig. 1 bis Fig. 4. Die FcεRIα-(His)₆-beschichtete Festphasenoberfläche bildet dabei in Variante B) die Komponente 1 eines Nachweissets ("Kit") für den Nachweis einer parasitären Infektion; deren Bereitstellung stellt den entsprechenden ersten Schritt des Verfahrens dar.

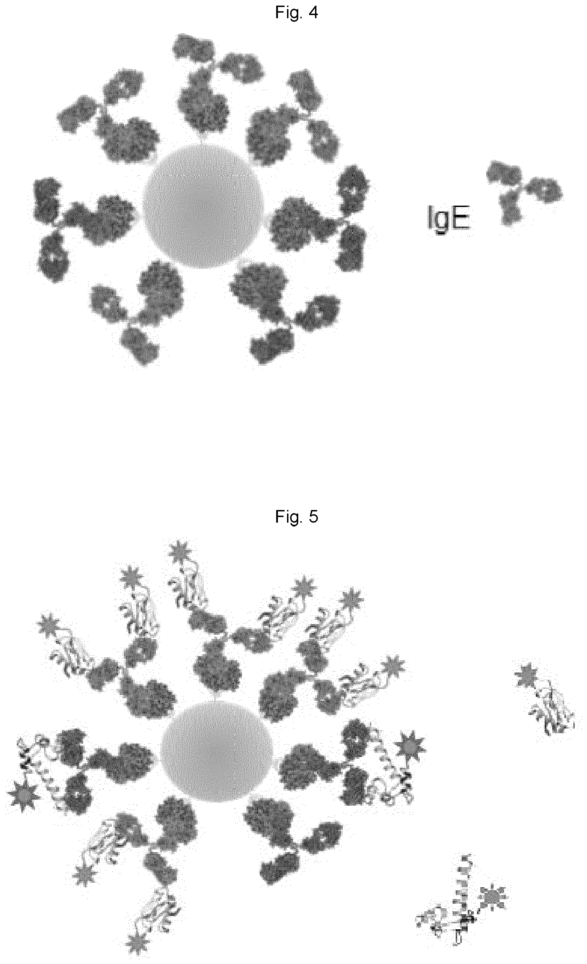

Der nächste Schritt des Verfahrens zum Nachweis einer Parasiten-Infektion umfasst die Inkubation der FcεRIα-(His)₆-beschichteten Festphasenoberfläche mit der zu untersuchenden biologischen Probe, was zu der in Fig. 4 beispielhaft symbolisch dargestellten Situation führt. Die mit dem hochaffinen IgE-Rezeptor FcεRIα-(His)₆ beschichtete Festphasenoberfläche bindet dabei sämtliche in der biologischen Probe vorhandenen IgE-Varianten, und zwar so lange, bis alle IgE-Rezeptoren belegt sind (bei einem Überschuß an IgE).

Nun folgt auch hier ein Waschschritt, um die störenden Begleitstoffe aus der biologischen Probe zu entfernen.

Im nächsten Schritt wird die mit IgE aus der biologischen Probe beladene Festphasenoberfläche, d.h. die IgE-beschichtete Festphasenoberfläche, mit mindestens einem bekannten markierten parasitären Allergen inkubiert sowie anschließend wieder gewaschen, um einen eventuellen Überschuss des mindestens einen bekannten markierten Allergens zu entfernen (vgl. Fig. 5). Dieses mindestens eine bekannte markierte Allergen ist diagnostisch validiert und dient in dieser Ausgestaltung des erfindungsgemäßen Verfahrens als Reagenz für den Infektionsnachweis; es stellt in der entsprechenden direkt einsetzbaren Applikationsform beispielsweise die zweite Komponente eines gebrauchsfertigen Nachweis-Sets ("Kit") dar. Wenn der Patient mit dem Parasiten infiziert ist (oder kürzlich war), von dem das mindestens eine bekannte markierte parasitäre Allergen ursprünglich/natürlicherweise stammt, dann befindet sich auf der IgE-beschichteten Festphasenoberfläche auch dasjenige Immunglobulin E, das spezifisch gegen das bekannte parasitäre Allergen gerichtet ist, und infolge der spezifischen Bindung in nativer Form von IgE und bekanntem markierten Allergen (bzw. dessen konformationellem Epitop), wird das bekannte markierte Allergen selektiv an die IgE-beschichtete Festphasenoberfläche gebunden und damit fixiert. Anhand der Markierung des bekannten parasitären Allergens wird dessen Anwesenheit auf der gewaschenen, nun spezifisch zumindest bereichsweise auch mit dem bekannten markierten parasitären Allergen-beschichteten Festphasenoberfläche nachgewiesen, sofern das entsprechende Allergen-spezifische IgE in der biologischen Probe enthalten war. Mit dem Nachweis der Markierung auf der wie vorstehend beschrieben behandelten Festphasenoberfläche ist somit eine entsprechende parasitäre Infektion nachgewiesen. Dem Fachmann sind aus dem Stand der Technik zahlreiche Markierungsmethoden sowie deren Nachweistechniken bekannt, die sämtlich erfindungsgemäß eingesetzt werden können, beispielsweise Isotopen-Markierungen (sowohl mit radioaktiven Isotopen als auch mit nicht-radioaktiven Isotopen), Markierungen mit Hilfe von chemisch an das Allergen angebundenen Chromophoren und/oder Fluorophoren u.v.a.m. Der Nachweis kann beispielsweise mittels Fluoreszenzmessung in einem sog. Plate Reader erfolgen. Die mit dem mindestens einen bekannten markierten Allergen inkubierte Festphasenoberfläche (z. B. Mikropartikel) ist bei Anwesenheit von parasiten-spezifischen IgEs im Serum oder im Plasma beispielsweise fluoreszierend markiert und zeigt einen positiven Infektionsstatus an. Durch Kombination verschiedener miteinander kompatibler Fluorophore lassen sich IgE Antikörper mit Spezifitäten für mehrere Allergene, aus dem gleichen oder verschiedenen pathogenen Organismen, gleichzeitig nachweisen.

Für den Nachweis der indirekten Bindung des markierten bekannten Allergens an die Festphasenoberfläche via IgE (und damit einer vorliegenden Infektion) kann das markierte bekannte Allergen auch von der Festphasenoberfläche abgelöst und dann erst - beispielsweise in Lösung - nachgewiesen werden. Dies ist zwar nachteilig insofern als dafür mindestens ein weiterer Verfahrensschritt erforderlich ist, nämlich die Elution des markierten bekannten Allergens von der Festphasenoberfläche, aber es können bei diesem Vorgehen ggf. weitere Nachweisverfahren erfindungsgemäß eingesetzt werden, die auf Festphasen-adsorbiertes markiertes Allergen nicht oder nur schlecht anwendbar sind, beispielsweise NMR-Untersuchungen.

Es ist weiterhin erfindungsgemäß, mehrere in bekannter Weise unterschiedlich markierte bekannte Allergene gleichzeitig mit der IgE-beschichteten Festphase zu inkubieren, da damit eine parallele Bestimmung von Mehrfachinfektionen schnell und kostengünstig vorgenommen werden kann (vgl. Fig. 5). Als unterschiedliche nebeneinander nachweisbare Markierungen kann beispielsweise die Verwendung verschiedener Fluorophore und/oder Chromophore vorgesehen werden, oder die Kombination von Fluorophor/Chromophor mit radioaktiver Isotopenmarkierung.

Die Herstellung von geeigneten markierten Allergenen ist dem Fachmann bekannt, beispielsweise durch fermentative Prozesse mit anschließender chemischer Modifikation des fermentativ gewonnenen und aufgereinigten Allergens, so dass darauf hier nicht näher eingegangen werden muss. Alle im Stand der Technik bekannten Herstellungsverfahren können verwendet werden, um entsprechend markierte Allergene herzustellen, die dann erfindungsgemäß verwendet werden können.

Das erfindungsgemäße Verfahren zum Infektionsnachweis ist universell anwendbar, da das zentrale Merkmal, nämlich die Isolation von Infektionen anzeigenden IgE-Varianten grundsätzlich für alle IgE-Varianten funktioniert, da die Bindung an den IgE-Rezeptor FcεRIα-(His)₆ universeller Natur ist: Alle infektionsrelevanten Immunglobuline vom Typ E (IgE, bzw. Mehrzahl IgEs) werden vom FcεRIα-(His)₆-Rezeptor mit hoher Affinität und unter Erhalt der nativen Konformation gebunden. Es ist daher ohne weiteres ersichtlich, dass die Anwendung des Verfahrens mit beliebigen bekannten Allergenen in den Schutzumfang des Gegenstands der Erfindung fällt. Beispielhaft konkret benannt seien an dieser Stelle Infektionen mit Schistosomen, Echinokokken oder Spulwürmern (Ascaris-Arten).

Die Ausgestaltung der Erfindung als Verfahren zum Nachweis einer parasitären oder pilzlichen Infektion läßt sich daher wie folgt zusammenfassen:
Der Gegenstand der Erfindung als Verfahren zum Nachweis einer parasitären oder pilzlichen Infektion ist ein Verfahren zum in-vitro Nachweis von Antikörpern, die durch eine parasitäre oder pilzliche Infektion gebildet wurden, und ist dadurch gekennzeichnet, daß es folgende Verfahrensschritte umfasst:
a) Bereitstellung einer Festphasenoberfläche, die zumindest bereichsweise mit dem IgE Rezeptor FcεRIα-(His)₆ beladen ist, wobei die IgE-Bindungsstelle des IgE Rezeptors FcεRIα-(His)₆ in der Lage ist, IgE zu binden;
b) Inkubierung der gemäß Schritt a) bereitgestellten Festphasenoberfläche mit einer biologischen Probe, welche im Falle einer parasitären oder pilzlichen Infektion des Patienten, von dem die biologische Probe stammt, auch mindestens ein IgE enthält, das mindestens ein parasitäres oder pilzliches Allergen bindet, das im Falle einer parasitären oder pilzlichen Infektion des Patienten mindestens in der biologischen Probe vorhanden ist, wobei der an die Festphasenoberfläche gebundene IgE Rezeptor FcεRIα-(His)₆ seinerseits die in der biologischen Probe enthaltenen IgE bindet, so daß die aus der biologischen Probe stammenden IgE über den IgE Rezeptor FcεRIα-(His)₆ an die Festphasenoberfläche gebunden werden, wodurch eine Festphasenoberfläche erhalten wird, die vermittelst FcεRIα-(His)₆ als Anker mit IgE aus einer biologischen Probe beladen ist;
c) Waschen der gemäß Schritt b) erhaltenen, mit IgE beladenen Festphasenoberfläche;
d) Inkubation der gemäß Schritt c) erhaltenen mit IgE beladenen Festphasenoberfläche mit mindestens einem markierten parasitären oder pilzlichen Allergen, dessen natürliche, nicht markierte Form mit der parasitären oder pilzlichen Infektion ursächlich zusammenhängt, so daß das mindestens eine markierte parasitäre oder pilzliche Allergen über das dieses Allergen bindende IgE an die Festphasenoberfläche gebunden wird, was nur dann erfolgt, wenn das dieses Allergen bindende IgE in dem zu untersuchenden Serum oder Plasma während der Durchführung von Schritt b) vorhanden war;
e) Waschen der gemäß Schritt d) inkubierten Festphasenoberfläche, so daß nicht gebundene überschüssige Mengen des mindestens einen markierten parasitären oder pilzlichen Allergens entfernt werden, wodurch eine Festphasenoberfläche erhalten wird, die nur dann eine Markierung aufweist, wenn die zu untersuchende biologische Probe ein zu dem mindestens einen parasitären oder pilzlichen Allergen kompatibles IgE enthalten hat;
f) Untersuchung der gemäß Schritt e) gewaschenen Festphasenoberfläche mittels mindestens eines Verfahrens, das die Markierung des mindestens einen markierten parasitären oder pilzlichen Allergens nachweist, wobei nur dann ein positives Untersuchungsergebnis erhalten wird, und damit einen positiven Antikörper-Nachweis darstellt, wenn das zu diesem mindestens einen markierten parasitären Allergen kompatible IgE in der biologischen Probe enthalten war.

Der Gegenstand der Erfindung als Verfahren zum Nachweis einer parasitären oder pilzlichen Infektion ist in einer weiteren Ausgestaltung ein Verfahren wie vorstehend beschrieben, das weiterhin dadurch gekennzeichnet ist, daß es sich bei der Festphasenoberfläche um eine makroskopische stationäre Festphase handelt, die entweder monolithisch ausgebildet ist oder aus einem Faserverbund besteht.

Der Gegenstand der Erfindung als Verfahren zum Nachweis einer parasitären oder pilzlichen Infektion ist in einer weiteren Ausgestaltung ein Verfahren wie vorstehend beschrieben, das weiterhin dadurch gekennzeichnet ist, daß die Festphasenoberfläche aus einem keramischen und/oder einem polymeren Material besteht.

Der Gegenstand der Erfindung als Verfahren zum Nachweis einer parasitären oder pilzlichen Infektion ist in einer weiteren Ausgestaltung ein Verfahren wie oben beschrieben, das im Gegensatz zur im vorherigen Absatz beschriebenen Ausgestaltung dadurch gekennzeichnet ist, daß es sich bei der Festphasenoberfläche um eine Festphasenoberfläche handelt, gebildet aus der Gesamtheit der Partikeloberflächen einer Menge von Partikeln, die gemeinsam den Verfahrensschritten a) bis f) unterzogen werden.

Der Gegenstand der Erfindung als Verfahren zum Nachweis einer parasitären oder pilzlichen Infektion ist in einer besonderen Ausgestaltung des Verfahrens gemäß vorstehendem Absatz, dadurch gekennzeichnet, daß es sich bei den Partikeln um magnetische Partikel handelt.

Der Gegenstand der Erfindung als Verfahren zum Nachweis einer parasitären oder pilzlichen Infektion ist in einer weiteren Ausgestaltung dadurch gekennzeichnet, daß es sich bei der Markierung des parasitären oder pilzlichen Allergens um eine Farbmarkierung und/oder um eine Fluoreszenzmarkierung und/oder um eine Isotopenmarkierung und/oder um eine radiologische Markierung handelt.

Der Gegenstand der Erfindung als Verfahren zum Nachweis einer parasitären oder pilzlichen Infektion ist in einer weiteren Ausgestaltung dadurch gekennzeichnet, daß es sich bei den Antikörpern, die durch eine parasitäre oder pilzliche Infektion gebildet wurden, um Antikörper handelt, die durch eine Infektion mit Parasiten gebildet wurden, ausgewählt aus der Liste umfassend die Parasiten Schistosomen, Spulwürmer, Echinococcus.

Der Gegenstand der Erfindung als Verfahren zum Nachweis einer parasitären oder pilzlichen Infektion ist in einer weiteren Ausgestaltung dadurch gekennzeichnet, daß es sich bei den Antikörpern, die durch eine parasitäre oder pilzliche Infektion gebildet wurden, um Antikörper handelt, die durch eine Infektion mit Pilzen gebildet wurden, ausgewählt aus der Liste umfassend die Pilze der Gattungen Alternaria, Cladosporium, Penicillium, Aspergillus, Malassezia.

Der Gegenstand der Erfindung als Verfahren zum Nachweis einer parasitären oder pilzlichen Infektion umfasst ein Kit zum Nachweis von Antikörpern nach einem Verfahren gemäß einer der vorstehend beschriebenen Ausführungsformen, das dadurch gekennzeichnet ist, daß es umfasst:
- eine Festphasenoberfläche, die wenigstens bereichsweise mit dem
   IgE Rezeptor FcεRIα-(His)₆ beladen ist;
- eine Reagenzlösung umfassend mindestens ein markiertes parasitäres
   Allergen und/oder mindestens ein markiertes pilzliches Allergen, wobei es sich bei der Markierung jeweils um Farbmarkierung und/oder Fluoreszenzmarkierung und/oder Isotopenmarkierung und/oder radiologische Markierung handelt.

### Ausführungsbeispiele

Ausführungsbeispiel 1:
Ausführungsbeispiel 1 beschreibt das Verfahren der Identifikation eines Allergens anhand seines konformationellen Epitops exemplarisch unter Verwendung des Grün fluoreszierenden Proteins (GFP) als Modell-Allergen für ein unbekanntes zu identifizierendes Allergen. Dieses wird aus einem komplexen Proteingemisch, einem Zellextrakt, selektiv mittels eines IgE-Antikörpers (rekombinantes anti-GFP IgE) erfindungsgemäß isoliert und identifiziert.

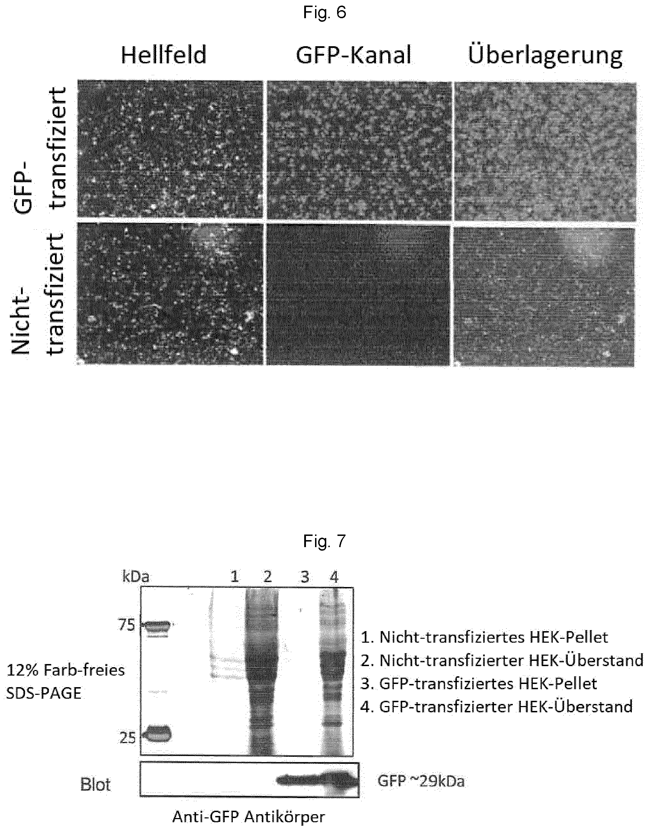

Dazu werden zunächst HEK293 Zellen transient mit einem GFP-kodierenden Plasmid transfiziert. Die Entstehung von GFP in den transfizierten Zellen wird mikroskopisch (Fig. 6) und durch SDS-PAGE/Western Blots (Fig. 7) experimentell bestätigt. 24 Stunden nach der Transfektion werden Extrakte der Zellen hergestellt. Als Kontrolle werden nicht transfizierte HEK293 Zellen verwendet. In diesem Beispiel ist die zu beschichtende Festphasenoberfläche die Summe der Oberflächen von magnetischen Beads, die gemeinsam den Verfahrensschritten unterzogen werden. Die zu wählenden Versuchsparameter für diese vorbereitenden Transfektionsversuche sind dem Fachmann bekannt; sie sind nicht Bestandteil des erfindungsgemäßen Verfahrens.

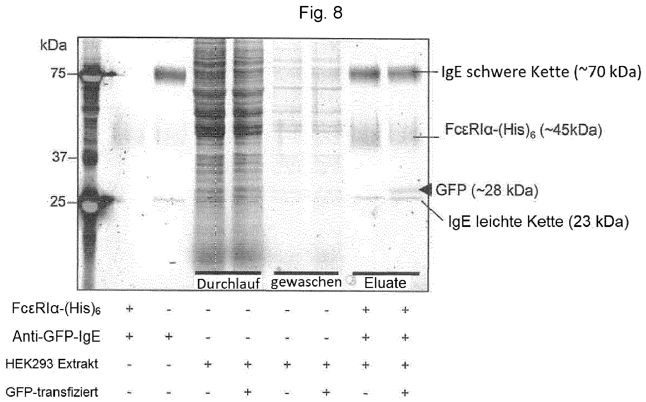

Die magnetischen Beads werden mit rekombinantem, hochaffinen IgE Rezeptor mit Histidin-Tag (FcεRIα-(His)₆) beladen und anschließend mit einem anti-GFP IgE Antikörper inkubiert. Die mit dem IgE-Antikörper beladenen magnetischen Beads werden gewaschen und die eine Hälfte von Ihnen mit einem Extrakt aus HEK293/GFPtransfizierten Zellen inkubiert, die andere Hälfte mit einem Extrakt aus nicht-transfizierten HEK293 Zellen. Ab hier oder spätestens nach dem folgenden Waschschritt werden die mit den verschiedenen Extrakten inkubierten magnetischen Beads in den nachfolgenden Schritten getrennt voneinander aber unter jeweils gleichen Bedingungen weiterverarbeitet. Im vorliegenden Beispiel werden sie zunächst gewaschen sowie anschließend mittels Imidazol jeweils ein Eluat hergestellt. In dem so gewonnenen Eluat werden dann die Proteine mittels SDS-PAGE aufgetrennt und das gefundene (Modell-)Allergen identifiziert, da nur dieses eine Bande in der exemplarisch verwendeten SDS-Page-Trennung ergibt, die mit dem Eluat aus den transfizierten Zellen durchgeführt wird, zu der es keine entsprechende Bande in der SDS-PAGE-Trennung mit dem Eluat aus den nicht transfizierten Referenzzellen gibt (Fig. 8). Dem Fachmann sind die für die Durchführung der vergleichenden SDS-PAGE-Trennungen erforderlichen Versuchsparameter bekannt, so daß darauf hier nicht näher eingegangen werden muß.

Ausführungsbeispiel 2:
Ausführungsbeispiel 2 beschreibt beispielhaft das Verfahren zum in-vitro Nachweis einer parasitären oder pilzlichen Infektion. Hierbei ist es unwesentlich, ob für den experimentellen Nachweis infektiöse Patientenseren oder Seren von Allergikern verwendet werden, da es sich in allen Fällen um eine vergleichbare Interaktion zwischen Allergen und Allergen-spezifischem IgE handelt. Die magnetischen Beads werden zunächst mit rekombinantem, hochaffinen IgE Rezeptor mit Histidin-Tag (FcεRIα-(His)₆) beladen und im nächsten Schritt mit einem rekombinanten IgE Antikörper, der ein spezifisches Erdnußallergen (z.B. Ara h 1 oder Ara h 2) erkennt, beladen. Nach Beladung mit dem IgE Antikörper werden die magnetischen beads mit den diagnostischen, fluoreszierend markierten Allergenen inkubiert. Nach einem Waschschritt zur Abtrennung ungebundener Komponenten wird die Fluoreszenz in einem Spektrofluorimeter gemessen. Erfindungsgemäß können auch Kombinationen von diagnostischen Allergenen zum Einsatz kommen, die mit fluoreszierenden Gruppen markiert sind, deren Absorptions- und Emissionsspektren nicht oder nur unwesentlich überlappen. In der diagnostischen Anwendung findet die Beladung mit dem Serum der Patienten statt. Falls im Patientenserum spezifisches IgE gegen das diagnostische Allergen enthalten ist, läßt sich dies anhand der nachfolgenden Fluoreszenzmessung quantitativ nachweisen.

### Abbildungslegenden und Bezugszeichenliste

- Fig. 1:: Beispielhaft symbolische Darstellung eines IgE-beschichteten mag-netischen Ni-NTA-Partikels.
- Fig. 2:: Beispielhaft symbolische Darstellung der selektiven Bindung des gesuchten (noch unbekannten) Allergens an die IgE-beschichtete Oberfläche eines magnetischen Ni-NTA-Partikels.
- Fig. 3:: Beispielhaft symbolische Darstellung einer FcεRIα-(His)₆-beschichteten Festphasenoberfläche, die die Komponente 1 eines Nachweissets für den Nachweis einer parasitären Infektion darstellt.
- Fig. 4:: Beispielhaft symbolische Darstellung einer FcεRIα-(His)₆-beschichteten Festphasenoberfläche während bzw. nach der Inkubation mit dem Serum im Zuge der Ausgestaltung der Erfindung als Nachweisverfahren einer parasitären Infektion.
- Fig. 5:: Beispielhaft symbolische Darstellung einer IgE-beschichteten Festphasenoberfläche während der Inkubation mit dem mindestens einen markierten bekannten Allergen im Zuge der Ausgestaltung der Erfindung als Nachweisverfahren einer parasitären Infektion. Die Mar-kierungen (beispielhaft Fluorophore) werden durch Sternsymbole dargestellt.
- Fig. 6:: Beispielhafte Mikroskopiebilder zum Nachweis der erfolgten GFP-Transfektion bei Ausführungsbeispiel 1.
- Fig. 7:: Beispielhafter SDS-PAGE/Western Blot-Nachweis der erfolgten GFP-Transfektion bei Ausführungsbeispiel 1.
- Fig. 8:: Beispielhafte SDS-PAGE-Auswertung von Ausführungsbeispiel 1.

Gleiche Bezugszeichen haben in den verschiedenen Figuren die gleiche Bedeutung.
- Ni²⁺-NTA:: Bezeichnung für ein Ni²⁺-Ion, gebunden (komplexiert) an Nitrilo-triessigsäure ("Nitrilotriacetic acid" = "NTA".
- IgE:: Immunglobuline vom Typ E, je nach vorliegender Variante in der Immunabwehr spezifisch wirkend gegen unterschiedlichste Allergene (jede einzelne IgE-Variante gegen ein spezifischen Allergen.
- SDS-PAGE:: Natriumdodecylsulfat-Polyacrylamidgelelektrophorese).
- 2D-PAGE:: zweidimensionale Gelelektrophorese (synonym: 2D-Gelelektrophorese).

## Patentansprüche

1. Verfahren zum in-vitro Nachweis von Antikörpern, die durch eine parasitäre oder pilzliche Infektion gebildet wurden, **dadurch gekennzeichnet, daß** es folgende Verfahrensschritte umfasst:
a) Bereitstellung einer Festphasenoberfläche, die zumindest bereichsweise mit dem IgE Rezeptor FcεRIα-(His)₆ beladen ist, wobei die IgE-Bindungsstelle des IgE Rezeptors FcεRIα-(His)₆ in der Lage ist, IgE zu binden;
b) Inkubierung der gemäß Schritt a) bereitgestellten Festphasenoberfläche mit einer biologischen Probe, welche im Falle einer parasitären oder pilzlichen Infektion des Patienten, von dem die biologische Probe stammt, auch mindestens ein IgE enthält, das mindestens ein parasitäres oder pilzliches Allergen bindet, das im Falle einer parasitären oder pilzlichen Infektion des Patienten mindestens in der biologischen Probe vorhanden ist, wobei der an die Festphasenoberfläche gebundene IgE Rezeptor FcεRIα-(His)₆ seinerseits die in der biologischen Probe enthaltenen IgE bindet, so daß die aus der biologischen Probe stammenden IgE über den IgE Rezeptor FcεRIα-(His)₆ an die Festphasenoberfläche gebunden werden, wodurch eine Festphasenoberfläche erhalten wird, die vermittelst FcεRIα-(His)₆ als Anker mit IgE aus einer biologischen Probe beladen ist;
c) Waschen der gemäß Schritt b) erhaltenen, mit IgE beladenen Festphasenoberfläche;
d) Inkubation der gemäß Schritt c) erhaltenen mit IgE beladenen Festphasenoberfläche mit mindestens einem markierten parasitären oder pilzlichen Allergen, dessen natürliche, nicht markierte Form mit der parasitären oder pilzlichen Infektion ursächlich zusammenhängt, so daß das mindestens eine markierte parasitäre oder pilzliche Allergen über das dieses Allergen bindende IgE an die Festphasenoberfläche gebunden wird, was nur dann erfolgt, wenn das dieses Allergen bindende IgE in dem zu untersuchenden Serum oder Plasma während der Durchführung von Schritt b) vorhanden war;
e) Waschen der gemäß Schritt d) inkubierten Festphasenoberfläche, so daß nicht gebundene überschüssige Mengen des mindestens einen markierten parasitären oder pilzlichen Allergens entfernt werden, wodurch eine Festphasenoberfläche erhalten wird, die nur dann eine Markierung aufweist, wenn die zu untersuchende biologische Probe ein zu dem mindestens einen parasitären oder pilzlichen Allergen kompatibles IgE enthalten hat;
f) Untersuchung der gemäß Schritt e) gewaschenen Festphasenoberfläche mittels mindestens eines Verfahrens, das die Markierung des mindestens einen markierten parasitären oder pilzlichen Allergens nachweist, wobei nur dann ein positives Untersuchungsergebnis erhalten wird, und damit einen positiven Antikörper-Nachweis darstellt, wenn das zu diesem mindestens einen markierten parasitären Allergen kompatible IgE in der biologischen Probe enthalten war.

2. Verfahren gemäß Anspruch 1, **dadurch gekennzeichnet, daß** es sich bei der Festphasenoberfläche um eine makroskopische stationäre Festphase handelt, die entweder monolithisch ausgebildet ist oder aus einem Faserverbund besteht.

3. Verfahren gemäß Anspruch 2, **dadurch gekennzeichnet, daß** die Festphasenoberfläche aus einem keramischen und/oder einem polymeren Material besteht.

4. Verfahren gemäß Anspruch 1, **dadurch gekennzeichnet, daß** es sich bei der Festphasenoberfläche um eine Festphasenoberfläche handelt, gebildet aus der Gesamtheit der Partikeloberflächen einer Menge von Partikeln, die gemeinsam den Verfahrensschritten a) bis f) unterzogen werden.

5. Verfahren gemäß Anspruch 4, **dadurch gekennzeichnet, daß** es sich bei den Partikeln um magnetische Partikel handelt.

6. Verfahren gemäß einem der Ansprüche 1 bis 5, **dadurch gekennzeichnet, daß** es sich bei der Markierung des parasitären oder pilzlichen Allergens um eine Farbmarkierung und/oder um eine Fluoreszenzmarkierung und/oder um eine Isotopenmarkierung und/oder um eine radiologische Markierung handelt.

7. Verfahren gemäß einem der Ansprüche 1 bis 6, **dadurch gekennzeichnet, daß** es sich bei den Antikörpern, die durch eine parasitäre oder pilzliche Infektion gebildet wurden, um Antikörper handelt, die durch eine Infektion mit Parasiten gebildet wurden, ausgewählt aus der Liste umfassend die Parasiten Schistosomen, Spulwürmer, Echinococcus.

8. Verfahren gemäß einem der Ansprüche 1 bis 6, **dadurch gekennzeichnet, daß** es sich bei den Antikörpern, die durch eine parasitäre oder pilzliche Infektion gebildet wurden, um Antikörper handelt, die durch eine Infektion mit Pilzen gebildet wurden, ausgewählt aus der Liste umfassend die Pilze der Gattungen Alternaria, Cladosporium, Penicillium, Aspergillus, Malassezia.

9. Kit zum Nachweis von Antikörpern nach einem Verfahren gemäß einem der Ansprüche 1 bis 8, **dadurch gekennzeichnet, daß** es umfasst:
- eine Festphasenoberfläche, die wenigstens bereichsweise mit dem IgE Rezeptor FcεRIα-(His)₆ beladen ist;
- eine Reagenzlösung umfassend mindestens ein markiertes parasitäres Allergen und/oder mindestens ein markiertes pilzliches Allergen, wobei es sich bei der Markierung jeweils um Farbmarkierung und/oder Fluoreszenzmarkierung und/oder Isotopenmarkierung und/oder radiologische Markierung handelt.
